# EUROPEAN PATENT APPLICATION

(11) **EP 4 792 826 A1**
(43) Date of publication of application: **19.08.2026**
(21) Application number: 24876667.7
(22) Date of filing: 12.10.2024
(51) Int. Cl.: A61K 38/18, C07K 14/48, A61P 25/28

(54) **NGF-FC FUSION PROTEIN FORMULATION AND USE THEREOF**

(30) Priority: 12.10.2023 WO PCT/CN2023/124142
(71) Applicant: Staidson (Beijing) Biopharmaceuticals Co., Ltd., Beijing 100176 (CN)
(72) Inventor: LIAO, Chuan, Beijing 100176 (CN); LI, Qinglei, Beijing 100176 (CN); WANG, Jia, Beijing 100176 (CN)
(74) Representative: WSL Patentanwälte Partnerschaft mbB
(86) International application number: PCT/CN2024/124343
(87) International publication number: WO 2025/077862

(57) **Abstract**

An NGF-Fc fusion protein formulation. The formulation has high stability under high-temperature, illumination, oscillation, freezing and thawing, acceleration and long-term conditions, thus ensuring good stability of the formulation during preparation, transportation and storage, and ensuring clinical medication safety and quality controllability.

## Description

### FIELD OF THE INVENTION

The present application relates to the field of biomedicine technology, and especially the NGF-Fc fusion protein formulation and uses thereof.

### BACKGROUND OF THE INVENTION

Nerve Growth Factor (NGF) is the first discovered member of the neurotrophin family, found in mouse sarcoma cells by Italian scientist Levi-Montlcini in 1953. NGF plays an important regulatory role in the development, differentiation, growth, regeneration, and expression of functional properties of central and peripheral neurons. NGF has been used to treat various neurological developmental disorders, including amblyopia, neuroma, various nerve injuries, and neurological diseases. However, its side effects such as causing pain, a short half-life in vivo, the limitation of low-dose administration to avoid pain sensitivity and frequent administration have restricted the wide application of NGF. Fusing protein drugs with longer half-lives and/or larger molecular weights components is a strategy to make some protein drugs have long-lasting activity.

However, there are very few Fc fusion protein drug formulations on the market, and no publicly available NGF-Fc fusion protein formulations have been disclosed. The application obtains an NGF-Fc fusion protein formulation with high stability through reasonable design, screening prescription and detection, the fusion protein formulation can be used for preparing injection and eye drops, and the fusion protein formulation can ensure good stability of the formulation during preparation, transportation and storage, and ensure quality controllability and clinical medication safety.

### DESCRIPTION OF THE APPLICATION

In one aspect, the present application provides an NGF-Fc fusion protein formulation, the formulation comprises an NGF-Fc fusion protein, a stabilizer, a surfactant, an antioxidant and a buffer solution, wherein, the stabilizer is trehalose or sucrose, the antioxidant is methionine, the surfactant is poloxamer, and the stabilizer is histidine buffer.

In some embodiments, the concentration of the fusion protein is from 0.05mg/ml to 5.0mg/ml, preferably from 0.05mg/ml to 2.0mg/ml, and more preferably from 0.1mg/ml to 0.5mg/ml. In some specific embodiments, the concentration of the fusion protein is 0.05mg/ml, 0.06mg/ml, 0.08mg/ml, 0.09mg/ml, 0.10mg/ml, 0.11mg/ml, 0.12mg/ml, 0.14mg/ml, 0.16mg/ml, 0.18mg/ml, or 2.0mg/ml.

In some embodiments, the concentration of the histidine buffer is from 8mM to 50mM, preferably from 10mM to 30mM. In some specific embodiments, the concentration of the histidine buffer is 10mM, 15mM, 20mM, 25mM, or 30mM.

In some embodiments, the concentration of the sucrose or the trehalose is from 6% to 12%, preferably from 7% to 10%. In some specific embodiments, the concentration of the sucrose or the trehalose is 7%, 7.5%, 8%, 8.5%, 9%, 9.5%, or 10%.

In some embodiments, the concentration of the methionine is from 0.05mM to 7mM, preferably from 0.1mM to 5mM, and more preferably from 1mM to 3mM. In some specific embodiments, the concentration of the methionine is 0.1mM, 0.5mM, 1 mM, 1.5mM, 2 mM, 2.5mM, 3 mM, 3.5mM, 4 mM, 4.5mM, or 5 mM.

In some embodiments, the concentration of the poloxamer is from 0.005% to 0.3%, preferably 0.01% to 0.2%, and more preferably 0.01% to 0.03%. In some specific embodiments, the concentration of the poloxamer is 0.01%, 0.02%, 0.03%, 0.04%, 0.06%, 0.08%, 0.1%, 0.12%, 0.14%, 0.16%, 0.18% or 0.2%.

In some embodiments, the pH of the formulation is from 5.0 to 6.5, preferably from 5.0 to 6.2, more preferably from 5.0 to 5.4. In some specific embodiments, the pH of the formulation is 5.0, 5.1, 5.2, 5.3, 5.4, 5.6, 5.8, 6.0, or 6.2.

In some embodiments, the concentration of the fusion protein is from 0.05mg/ml to 2.0mg/ml, the concentration of the histidine buffer is from 10mM to 30mM, the concentration of the sucrose or the trehalose is from 7% to 10%, the concentration of the methionine is from 0.1mM to 5Mm, the concentration of the poloxamer is from 0.01% to 0.2%, and the pH of the formulation is from 5.0 to 6.2.

In some embodiments, the formulation further comprises a cyclodextrin, preferably hydroxypropyl beta cyclodextrin.

In some embodiments, the concentration of the cyclodextrin is from 0.05mM to 8mM, preferably from 1mM to 6mM, and more preferably 5 mM. In some specific embodiments, the concentration of the cyclodextrin is 1 mM, 2 mM, 3 mM, 4 mM, 5 mM, or 6 mM.

In some embodiments, the formulation is any one of the following formulations:
(1) the concentration of the fusion protein is 0.05mg/ml, 0.06mg/ml, 0.08mg/ml, 0.09mg/ml, 0.10mg/ml, 0.11mg/ml, 0.12mg/ml, 0.14mg/ml, 0.16mg/ml, 0.18mg/ml or 2.0mg/ml, the concentration of the histidine buffer is from 10mM to 30mM, the concentration of the sucrose or the trehalose is from 7% to 10%, the concentration of the methionine is from 0.1mM to 5Mm, the concentration of the poloxamer is from 0.01% to 0.2%, and the pH of the formulation is from 5.0 to 6.2;
(2) the concentration of the fusion protein is from 0.05mg/ml to 2.0mg/ml, the concentration of the histidine buffer is 10mM, 15mM, 20mM, 25mM or 30mM, the concentration of the sucrose or the trehalose is from 7% to 10%, the concentration of the methionine is from 0.1mM to 5Mm, the concentration of the poloxamer is from 0.01% to 0.2%, and the pH of the formulation is from 5.0 to 6.2;
(3) the concentration of the fusion protein is from 0.05mg/ml to 2.0mg/ml, the concentration of the histidine buffer is from 10mM to 30mM, the concentration of the sucrose or the trehalose is 7%, 7.5%, 8%, 8.5%, 9%, 9.5% or 10%, the concentration of the methionine is from 0.1mM to 5Mm, the concentration of the poloxamer is from 0.01% to 0.2%, and the pH of the formulation is from 5.0 to 6.2;
(4) the concentration of the fusion protein is from 0.05mg/ml to 2.0mg/ml, the concentration of the histidine buffer is from 10mM to 30mM, the concentration of the sucrose or the trehalose is from 7% to 10%, the concentration of the methionine is 0.1mM, 0.5mM, 1mM, 1.5mM, 2mM, 2.5mM, 3mM, 3.5mM, 4mM, 4.5mM or 5mM, the concentration of the poloxamer is from 0.01% to 0.2%, and the pH of the formulation is from 5.0 to 6.2;
(5) the concentration of the fusion protein is from 0.05mg/ml to 2.0mg/ml, the concentration of the histidine buffer is from 10mM to 30mM, the concentration of the sucrose or the trehalose is from 7% to 10%, the concentration of the methionine is from 0.1mM to 5mM, the concentration of the poloxamer is 0.01%, 0.02%, 0.03%, 0.04%, 0.06%, 0.08%, 0.1%, 0.12%, 0.14%, 0.16%, 0.18% or 0.2%, and the pH of the formulation is from 5.0 to 6.2;
(6) the concentration of the fusion protein is from 0.05mg/ml to 2.0mg/ml, the concentration of the histidine buffer is from 10mM to 30mM, the concentration of the sucrose or the trehalose is from 7% to 10%, the concentration of the methionine is from 0.1mM to 5mM, the concentration of the poloxamer is from 0.01% to 0.2%, and the pH value of the formulation is 5.0, 5.1, 5.2, 5.3, 5.4, 5.6, 5.8, 6.0 or 6.2.

In some embodiments, the formulation is any one of the following formulations:
(1) the concentration of the fusion protein is 0.1mg/ml, the concentration of the histidine buffer is 10mM, the concentration of the sucrose or the trehalose is 7%, the concentration of the methionine is 1.5mM, the concentration of the poloxamer is 0.01%, and the pH of the formulation is 5.4;
(2) the concentration of the fusion protein is 0.1mg/ml, the concentration of the histidine buffer is 20mM, the concentration of the sucrose or the trehalose is 9%, the concentration of the methionine is 1.0mM, the concentration of the poloxamer is 0.03%, and the pH of the formulation is 5.2;
(3) the concentration of the fusion protein is 0.5mg/ml, the concentration of the histidine buffer is 30mM, the concentration of the sucrose or the trehalose is 10%, the concentration of the methionine is 3mM, the concentration of the poloxamer is 0.02%, and the pH of the formulation is 5.0;
(4) the concentration of the fusion protein is 2.0mg/ml, the concentration of the histidine buffer is 20mM, the concentration of the sucrose or the trehalose is 8%, the concentration of the methionine is 2.0mM, the concentration of the poloxamer is 0.1%, and the pH of the formulation is 5.8;
(5) the concentration of the fusion protein is 0.5mg/ml, the concentration of the histidine buffer is 20mM, the concentration of the sucrose or the trehalose is 10%, the concentration of the methionine is 0.1mM, the concentration of the poloxamer is 0.2%, and the pH of the formulation is 6.2;
(6) the concentration of the fusion protein is 0.05mg/ml, the concentration of the histidine buffer is 20mM, the concentration of the sucrose or the trehalose is 8.5%, the concentration of the methionine is 5.0mM, the concentration of the poloxamer is 0.04%, and the pH of the formulation is 5.2.

In some embodiments, the concentration of the cyclodextrin is 1 mM, 2 mM, 3 mM, 4 mM, 5 mM or 6 mM.

In some embodiments, the formulation can also comprise a preservative. The preservative comprises, but is not limited to, stearyldimethylbenzylammonium chloride, hexamethylammonium chloride, benzalkonium chloride, benzethonium chloride, phenol, butanol or benzyl alcohol, alkyl parahydroxybenzoate, etc..

In some embodiments, the formulation is a liquid formulation, and preferably the formulation is an injection formulation or an eye drop formulation.

In some embodiments, the formulation is an eye drop formulation, and the formulation further comprises a viscosity modifier, preferably the viscosity modifier is hydroxypropyl methylcellulose; and more preferably, the viscosity modifier is hydroxypropyl methylcellulose K4M.

In some embodiments, the concentration of the viscosity modifier is from 0.4% to 0.5%.

In some embodiments, the eye drop formulation comprises the following components: the concentration of the fusion protein is from 0.05mg/ml to 2.0gmg/ml, the concentration of the histidine buffer is from 10mM to 30mM, the concentration of the trehalose is from 7% to 10%, the concentration of the methionine is from 0.1mM to 5mM, the concentration of the poloxamer is from 0.01% to 0.2%, the concentration of the cyclodextrin is from 1mM to 6mM, the concentration of the hydroxypropyl methylcellulose is from 0.4% to 0.5%, and the pH of the formulation is from 5.0 to 6.2.

In some embodiments, the viscosity of the eye drop formulation is from 25cp to 50cp, preferably from 30cp to 45cp.

In some embodiments, the eye drop formulation comprises the following components: the concentration of the fusion protein is 0.1mg/ml, the concentration of the histidine buffer is 20mM, the concentration of the trehalose is 8.5%, the concentration of the methionine is 1.0mM, the concentration of the poloxamer is 0.03%, the concentration of the cyclodextrin is 5mM, the concentration of the hydroxypropyl methylcellulose is 0.4%, 0.45% or 0.5%, and the pH of the formulation is 5.2.

In some embodiments, the NGF-Fc fusion protein comprises an NGF moiety and an Fc moiety from N-terminus to C-terminus, the Fc moiety is derived from an Fc moiety of IgG or a mutant of an Fc moiety of IgG, and the mutant of the Fc moiety of IgG comprises a site mutation associated with ADCC/CDC activity.

In some embodiments, the NGF moiety is a human nerve growth factor, preferably a human nerve growth factor mutant.

In some embodiments, the human nerve growth factor mutant comprises the mutation site of Phe12Glu with reference to the amino acidic position set forth in wild-type human nerve growth factor; preferably, the human nerve growth factor comprises the amino acidic sequence of SEQ ID NO:2.

In some embodiments, the NGF moiety is fused to the Fc moiety via a polypeptide linker, the polypeptide linker comprises the amino acidic sequence of SEQ ID NO:3.

In some embodiments, the Fc moiety is derived from an Fc of IgG1 comprising the amino acidic sequence of SEQ ID NO:4.

In some embodiments, the NGF-Fc fusion protein comprises the amino acidic sequence of SEQ ID NO:1.

In another aspect, the present application discloses the use of the NGF-Fc fusion protein formulation in the preparation of a medicament for the treatment of NGF-related diseases.

In some embodiments, the NGF-related diseases is a neurological diseases; preferably, the neurological disease is selected from the group consisting of: neonatal hypoxic-ischemic encephalopathy, cerebral palsy, critical illness myopathy, nerve deafness, recurrent laryngeal nerve injury, traumatic brain injury, tooth nerve injury, cerebral stroke, Down syndrome, amyotrophic lateral sclerosis, multiple sclerosis, spinal muscular atrophy, diffuse brain injury, thymus dysplasia, optic contusion, follicular dysplasia, spinal cord injury, glaucoma, neurotrophic keratitis, optic injury, neuromyelitis optica, retinal associated diseases, urinary incontinence, Alzheimer's disease, Parkinson's disease, Huntington's disease, dementia, Hypertensive Intracerebral Hemorrhage Neurological Dysfunction, Cerebral Small Vessel Disease, Acute Ischemic Stroke, corneal endothelial dystrophy, diabetic neuropathy, diabetic foot ulcer, neurogenic skin ulcer, pressure sore, neurotrophic corneal ulcer, diabetic corneal ulcer, and macular hole.

In some embodiments, the NGF-related disease is a non-neurological disease; preferably, the non-neurological disease is selected from the group consisting of: atrophy of spleen, contusion of spleen, diminished ovarian reserve, premature ovarian failure, Ovarian Hyperstimulation Syndrome, ovarian remnant syndrome, ovarian follicular dysplasia, spermatogenesis disorder such as oligozoospermia, asthenospermia, and oligoasthenospermia, ischemic ulcer, stress ulcer, rheumatoid ulcer, liver fibrosis, corneal ulcer, burns, oral ulcer, and venous leg ulcers.

The NGF-Fc fusion protein formulation provided by the present application has the following excellent effects:
1. The present application had identified the optimal formulation of the NGF-Fc fusion protein. Based on the formulation, it can be used for both the preparation of injection and eye drop. The formulation of the present application can significantly reduce the formation of protein aggregates in the formulation, slow down the change of charge heterogeneity, and maintain the good stability of the fusion protein formulation.
2. In the the NGF-Fc fusion protein formulation of the present application, hydroxypropyl methylcellulose is added as a viscosity modifier, and it can be used to prepare eye drop, ensuring that the eye drop has a viscosity suitable for intraocular application, and also maintaining the osmotic pressure of the eye drop formulation at approximately 300 mOsm/kg, which is close to the osmotic pressure of the internal environment of the human eye.
3. The injection and eye drop formulations of the present application have strong stability and can exhibit excellent stability under conditions of vibration, freeze-thaw, acceleration, and long-term, which can ensure the formulations to maintain good stability during preparation, transportation, and storage, and ensure the clinical medication safety and the quality controllability.

### EXAMPLES

The following provides a further description of the specific embodiments of the present application based on the examples. The advantages and characteristics of the application will become clearer with the description proceeds. However, these embodiments are illustrative only and do not constitute any limitation on the scope of the applicaiton. It should be understood by those skill in the art that the details and form of the technical solution of the application may be modified or replaced without departing from the spirit and scope of the application, but these modifications and substitutions fall within the scope of the protection of the application.

If the specific conditions or methods are not specified in the embodiments, the implementation shall be carried out according to the conventional conditions or methods. If the manufacturer of the reagents or instruments is not specified, they are all common products that can be purchased from the market.

The NGF-Fc fusion protein used in the examples of the present application is disclosed in the international patent application WO2022/105847A1, and the disclosure content is incorporated herein by reference.

The 2-118-L3Fc10-M3-5 polypeptide structure used in the examples of the present application is shown in Table 1:

**Table 1. NGF-Fc fusion protein sequence**

| Fusion protein | Mature β-NGF moiety | Linker | Fc moiety |
|---|---|---|---|
| 2-118-L3Fc10-M3-5 (mature SEQ ID NO: 1) | Mutant β-NGF 118aa (SEQ ID NO: 2) | (G4S)₃ (SEQ ID NO: 3) | IgG1 M3-5 (N' minus 5aa) (SEQ ID NO: 4) |

### Example 1. Screening of prescription composition of NGF-Fc fusion protein formulation

### I. Screening of surfactants and antioxidants

### 1. Design of prescriptions and formulation of preparations

NGF-Fc fusion protein 2-118-L3Fc10-M3-5 was taken, and sample of each prescription was prepared according to table 2, wherein the concentration of the fusion protein was 0.1mg/ml.

### 2. Stability test method

The samples just prepared were used as the sample at 0 hous. Subsequently, each sample was placed at -70°C±10°C, 25°C±2°C, 5°C±3°C for 1 month and 40°C±3°C for 2 weeks. After the placement was completed, the stability of each sample was tested according to the following methods.

The test methods were as follows: the appearance of the solution was observed visually; the concentration of the protein was detected by Titer method; the aggregation characteristic of the protein was detected by SEC-HPLC; and the charge heterogeneity was detected by WCX.

**Table 2. Prescription design for surfactant and antioxidant screening**

| | Prescription design | |
|---|---|---|
| 1 | | - |
| 2 | | 0.01% PS80 |
| 3 | | 0.01% PS20 |
| 4 | 20 mM histidine, 140mM arginine, pH6.0 | 0.01% P188 |
| 5 | | 0.01%P188+0.02%PS20 |
| 6 | | 1mM Met |
| 7 | | 1mM Met+0.01%P188 |
| 8 | | 1mM Met+0.01% PEG6000 |
| 9 | | 1mM Met+0.01%P188+0.02%PS20 |
| 10 | | 1mM Met+0.01%P188+0.02% PEG6000 |

### 3. Stability test results

### (1) Appearance and protein concentration

After the samples of the above formulations being placed at -70°C±10°C, -20°C±10°C and 5°C±3°C for 1 month, the concentration of fusion protein did not show any significant decrease, and the results were basically consistent among all groups.

However, in terms of appearance, after being placed at the -70°C±10°C, -20°C±10°C, 5°C±3°C conditions for 1 month, prescriptions 2, 3 and 5 showed visible insoluble particles, while the other groups did not have any visible foreign matter.

After being placed at the high-temperature 40°C±3°C conditions for 2 weeks, only the groups of prescription 7 and 10 showed no visible foreign matter, while the other groups of samples showed visible insoluble particles, mainly in the form of white powdery, short fibrous or sheet-like protein aggregate, which might be due to the disruption of the NGF-Fc fusion protein structure and led to insoluble particles and even precipitation.

The above result initially indicated that high-temperature had a destructive effect on NGF-Fc fusion protein, however the formulation of prescription 7 and 10 can provide better protection effect for fusion protein than the others.

### (2)Aggregate detection

The aggregation of each formulation was detected by SEC-HPLC, the results were shown in Table 3.

The results showed that at 0 hous, the main peak percentage by SEC-HPLC in prescriptions 2,3 and 5 were significantly lower, being 77.81%, 91.26% and 92.36% respectively, which indicated that the protein structure of the groups of prescription 2,3 and 5 had been damaged after they were prepared into liquid preparation. In contrast, the main peak percentage of other groups were more than 98%.

As mentioned above, after being placed at -70°C, -20°C, and 5°C for 1 month, the samples of prescriptions 2, 3, and 5 showed visible insoluble particles and significant precipitation, so SEC-HPLC testing was not carried out. The main protein peak percentage of all the other group was all above 97%, with a decrease of only 0.05% - 2.37% compared with 0h.

After being placed at a temperature of 40°C for 2 weeks, prescriptions 2, 3, and 5 showed a large amount of precipitation and were not detected by SEC-HPLC. In the other prescription groups, the main peak percentage decreased. The main peak percentage of prescriptions 4, 6-8, and 10 remained above 95% under each testing conditions, and the decrease being only 2.4% - 3.2%; the main peak percentage by SEC-HPLC of 9 group decreased to 90.25%, with a decrease of up to 9.27%.

The above results indicated that the addition of poly-olefin surfactants in the formulation can causes aggregation of the NGF-Fc fusion protein. In contrast, the addition of surfactant P188 provides a better protective effect for the NGF-Fc fusion protein.

**Table 3. The detection results by SEC-HPLC for NGF-Fc fusion protein formulation**

| Preparation | The the main peak by SEC-HPLC (%) | | | | | |
|---|---|---|---|---|---|---|
| | 0h | -70°C for 1 month | -20°C for 1 month | 5°C for 1 month | 40°C for 2 weeks | Changes at 40°C for 2 weeks compared with 0h (Δ) |
| 1 | 98.80 | 99.11 | 98.61 | 98.15 | 93.61 | -5.19 |
| 2 | 77.81 | - | - | - | - | - |
| 3 | 91.26 | - | - | - | - | - |
| 4 | 99.42 | 99.03 | 99.09 | 99.16 | 96.23 | -3.19 |
| 5 | 92.36 | - | - | - | - | - |
| 6 | 99.07 | 98.29 | 99.35 | 98.99 | 95.87 | -3.20 |
| 7 | 99.39 | 99.12 | 99.05 | 99.12 | 96.99 | -2.40 |
| 8 | 99.10 | 99.02 | 99.04 | 98.87 | 95.99 | -3.11 |
| 9 | 99.52 | 98.61 | 98.23 | 97.15 | 90.25 | -9.27 |
| 10 | 99.30 | 99.25 | 99.01 | 99.04 | 96.56 | -2.74 |

(3) 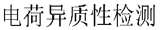

### (3) Charge heterogeneity detection

The charge heterogeneity of each formulation was detected by WCX, and the results were showen in Tables 4-6.

The results showed that at 0 hous, the main peak percentage by WCX in each group ranged from 54.19% to 59.25%, and the percentage of the acidicic peak and the basic peak were respectively in the range of 13.39% to 14.63% and 26.87% to 32.42%, respectively, which was basically the same among the groups.

After being placed at 70°C, -20°C and 5°C for 1 month, and at 40°C for 2 weeks, as previously mentioned, the samples in groups 2, 3 and 5 of the prescription were not tested because of precipitation.

In the remaining prescription groups, the percentage of the main peak, acidicic peak and basic peak in prescriptions 4, 6-8 and 10 did not change significantly after being placed at -70°C, -20°C and 5°C for 1 month, indicating that under the above conditions, the formulations of prescriptions 4, 6-8 and 10 also had excellent stability effect in terms of charge heterogeneity. In contrast, the main peak percentage by WCX in the samples of prescription 1 and 9 showed a downward trend, and the percentage of acidicic peak and basic peak also increased.

After being stored at 40°C for 2 weeks, the main peak percentage of the remaining prescriptions decreased, especially prescription 9, which decreased by 28.11% compared with that at 0, while prescriptions 4, 6-8, and 10 decreased by only about 19-20%, wherein prescription 7 decreased by only 14.73%, showing the best stability in all groups. The percentage of acidic peak and basic peak in each prescription increased. In contrast, prescription 7 performed best, with an increase of 11.82% in acidic peak (relative to 0), and only 2.91% in basic peak (relative to 0).

**Table 4. The main peak detection results by WCX for NGF Fc fusion protein formulation**

| Prescription | The percentage of main peak by WCX (%) | | | | | |
|---|---|---|---|---|---|---|
| | 0h | -70°C for 1 month | -20°C for 1 month | 5°C for 1 month | 40°C for 2 weeks | Changes at 40°C after 2 weeks compared with 0h (Δ) |
| 1 | 57.40 | 55.05 | 54.15 | 52.23 | 34.50 | -22.90 |
| 2 | 55.95 | - | - | - | - | - |
| 3 | 54.19 | - | - | - | - | - |
| 4 | 58.27 | 58.50 | 59.86 | 58.31 | 39.23 | -19.04 |
| 5 | 58.9 | - | - | - | - | - |
| 6 | 57.23 | 55.23 | 56.25 | 56.19 | 37.81 | -19.42 |
| 7 | 59.25 | 58.38 | 58.15 | 59.01 | 44.52 | -14.73 |
| 8 | 57.32 | 57.84 | 56.23 | 55.98 | 37.54 | -19.78 |
| 9 | 58.32 | 54.23 | 52.19 | 50.23 | 30.21 | -28.11 |
| 10 | 57.19 | 56.98 | 57.01 | 56.78 | 36.53 | -20.66 |

**Table 5. The acidic peak detection results by WCX for NGF Fc fusion protein formulation**

| Prescription | The percentage of acidic peak by WCX (%) | | | | | |
|---|---|---|---|---|---|---|
| | 0h | -70°C for 1 month | -20°C for 1 month | 5°C for 1 month | 40°C for 2 weeks | Changes at 40°C after 2 weeks compared with 0h (Δ) |
| 1 | 14.15 | 15.25 | 17.02 | 18.12 | 26.00 | 11.85 |
| 2 | 14.63 | - | - | - | - | - |
| 3 | 13.39 | - | - | - | - | - |
| 4 | 13.87 | 14.25 | 15.05 | 17.02 | 25.33 | 11.46 |
| 5 | 13.89 | - | - | - | - | - |
| 6 | 14.13 | 15.32 | 16.12 | 16.03 | 25.41 | 11.28 |
| 7 | 13.88 | 13.99 | 14.23 | 14.58 | 25.70 | 11.82 |
| 8 | 14.36 | 15.05 | 15.98 | 16.05 | 25.93 | 11.57 |
| 9 | 14.01 | 17.56 | 19.04 | 19.78 | 27.24 | 13.23 |
| 10 | 13.97 | 14.12 | 15.32 | 15.05 | 26.15 | 12.18 |

**Table 6. The basic peak detection results by WCX for NGF Fc fusion protein formulation**

| Prescription | The percentage of basic peak by WCX(%) | | | | | |
|---|---|---|---|---|---|---|
| | 0h | -70°C for 1 month | -20°C for 1 month | 5°C for 1 month | 40°C for 2 weeks | Changes at 40°C after 2 weeks compared with 0h(Δ) |
| 1 | 28.40 | 29.70 | 28.83 | 29.65 | 39.49 | 11.09 |
| 2 | 29.42 | - | - | - | - | - |
| 3 | 32.42 | - | - | - | - | - |
| 4 | 27.86 | 27.25 | 25.09 | 24.67 | 35.44 | 7.58 |
| 5 | 27.21 | - | - | - | - | - |
| 6 | 28.64 | 29.45 | 27.63 | 27.78 | 36.78 | 8.14 |
| 7 | 26.87 | 27.63 | 27.62 | 26.41 | 29.78 | 2.91 |
| 8 | 28.32 | 27.11 | 27.79 | 27.97 | 36.53 | 8.21 |
| 9 | 27.67 | 28.21 | 28.77 | 29.99 | 42.55 | 14.88 |
| 10 | 28.84 | 28.90 | 27.67 | 28.17 | 37.32 | 8.48 |

Based on the above results, for NGF Fc fusion protein formulation, the addition of polysorbate surfactants is easy to aggregate the fusion protein, while P188 and / or methionine show excellent protection. Therefore, the NGF Fc fusion protein of the present application selects P188 as the surfactant and methionine as the antioxidant, based on which, the next stage of prescription screening is carried out.

### II. Selection of buffers and stabilizers

### (1) Prescription design and formulation preparation

NGF Fc fusion protein 2-118-l3fc10-m3-5 was taken, and samples of each formulation were prepared according to table 7, and the concentration of fusion protein is 0.1mg/ml.

### 2. Stability test method

The samples just prepared were used as the samples at 0 hous. Subsequently, the samples were taken out under different influencing factors (accelerating conditions: 25°C±2°C, high-temperature conditions: 40°C±3°C) for 2 weeks, and the stability of each sample was tested.

The test methods were as follows: the appearance of the solution is observed visually; the osmotic pressure was detected by freezing point osmometer, the protein concentration was detected by Titer method; the aggregation characteristic of the protein was determined by SEC-HPLC, and the charge heterogeneity was determined by WCX.

**Table 7. Prescription design for the selection of buffers and stabilizers**

| Prescription | Composition | |
|---|---|---|
| 1 | 1mM Met, 0.01%P188 | pH5.2, 20mM Histidine buffer+140mM Arginine |
| 2 | | pH5.2, 20mM Histidine buffer+8% Sucrose |
| 3 | | pH5.2, 20mM Histidine buffer+8% Trehalose |
| 4 | | pH5.2, 20mM Histidine buffer+5% Mannitol |
| 5 | | pH7.0, 20mM Phosphate buffer+140mM Arginine |
| 6 | | pH7.0, 20mM Phosphate buffer+8% Sucrose |
| 7 | | pH7.0, 20mM Phosphate buffer+8% Trehalose |
| 8 | | pH7.0, 20mM Phosphate buffer+5% Mannitol |
| 9 | | pH5.2, 20mM Acetate buffer+140mM Arginine |
| 10 | | pH5.2, 20mM Acetate buffer+8% Sucrose |
| 11 | | pH5.2, 20mM Acetate buffer+8% Trehalose |
| 12 | | pH5.2, 20mM Acetate buffer+5% Mannitol |

### 3. Stability test results

### (1) Osmotic pressure, appearance, protein concentration

At 0 hous, the osmotic pressure of each prescription sample is 285mOsm/kg-299mOsm/kg; the pH of the prescriptions 1-4 and 9-12 is 5.15-5.38, and the pH of the prescriptions 5-8 is 6.84-6.86.

After storage for 2 weeks under accelerated and high-temperature conditions, the protein concentration of each prescription group did not change significantly.

After storage for 2 weeks under accelerated and high-temperature conditions, the appearance of the samples was observed by naked eyes, and it was found that only small white particles of protein aggregates appeared in prescriptions 1, 2 and 4; although the prescription 3 had slight opalescence, there were no visible insoluble particles; however, the prescriptions 5-12 all produced large flaky insoluble substances.

The results indicated that histidine buffer was more suitable for the stability of NGF-Fc fusion protein than phosphate buffer and acetate buffer.

### (2) Aggregate detection

The aggregation of each formulation was detected by SEC-HPLC, the results were shown in Table 8. Similarly, after storage for 2 weeks under accelerated and high-temperature conditions, due to the samlpes of prescription 5, 6, 8 groups precipitated seriously, aggregate detection was no longer carried out.

According to the test result in table 8, it can be seen that at 0 hours, the main peak percentage in each group was more than 98%, and there was basically no difference among the groups.

After 2 weeks under accelerated conditions, compared with 0h, the main peak percentage in prescriptions 1-4 had almost no change, while the main peak percentage by SEC-HPLC in prescription 7 and 9-12 decreased, but the range was only 1.58% - 2.20%. It is shown that under the above conditions, the above formulations can have good stability.

After 2 weeks under high-temperature, the main peak percentage in each group decreased compared with 0 hous. The main peak percentage by SEC-HPLC in prescriptions 1-4 decreased by 1.2% - 6.46%, and that in prescriptions 7 and 9-12 decreased by 4.42% - 9.14%. Among them, the main peak percentage in prescription 3 decreased by only 1.40%, showing the optimal stability.

**Table 8. The detection results by SEC-HPLC for NGF-Fc fusion protein formulation**

| Prescription | The percentage of main peak by SEC-HPLC(%) | | | | |
|---|---|---|---|---|---|
| | 0h | Accelerate for 2 weeks | Changes at acceleration for 2 weeks compared with 0h (Δ) | High-temperature for 2 weeks | Changes at high-temperature for 2 weeks compared with 0h (Δ) |
| 1 | 99.57 | 99.22 | -0.35 | 93.11 | -6.46 |
| 2 | 98.32 | 98.05 | -0.27 | 96.31 | -2.01 |
| 3 | 99.55 | 99.41 | -0.14 | 98.35 | -1.20 |
| 4 | 99.31 | 99.01 | -0.3 | 96.79 | -2.52 |
| 5 | 98.31 | - | - | - | - |
| 6 | 98.29 | - | - | - | - |
| 7 | 98.70 | 96.50 | -2.20 | 92.50 | -6.20 |
| 8 | 98.05 | - | - | - | - |
| 9 | 98.04 | 96.03 | -2.01 | 88.90 | -9.14 |
| 10 | 98.98 | 97.09 | -1.89 | 94.14 | -4.84 |
| 11 | 99.47 | 97.89 | -1.58 | 95.05 | -4.42 |
| 12 | 99.23 | 97.21 | -2.02 | 93.25 | -5.98 |

### (3) Charge heterogeneity detection

The charge heterogeneity of each formulation was detected by WCX and the results were shown in Tables 9-11. Similarly, after being stored for 2 weeks under accelerated and high-temperature conditions, the samples of prescriptions 5, 6, 8 precipitated seriously, the charge heterogeneity test was no longer carried out.

According to the test results in tables 9-11, it can be seen that at 0 hous, the main peak percentage of the samples in each prescription was in the range of 52.0% - 58.0%, and the percentage of the acidicic peak and the basic peak were between 12.7% - 15.5% and 27.1% - 35.2%, respectively, which was basically the same among the groups.

After 2 weeks under accelerated conditions, compared with 0h, the percentage of main peak, acidicic peak and basic peak in prescriptions 1-4 had almost no change, the decrease of the main peak percentage was less than 4.0%, and the increase of the acidicic peak percentage and the basic peak percentage was less than 3.0%. In contrast, the main peak percentage in the prescriptions 7 and 9-12 decreased by 6.1%-12.8%, the acidicic peak percentage increased by 2.5%-6.8%, and the basic peak percentage increased by 3.5%-8.5%. Among them, the main peak percentage of samples in the prescription 3 decreased by only 1.7%, and the acidicic peak percentage and the basic peak percentage had no significant change (≤ 1.0%), showing the best stability.

After 2 weeks under high-temperature, compared with 0h, the main peak percentage in each group decreased, the main peak percentage in the prescription 1-4 group decreased by 9.4% - 20.1%, and the main peak percentage in the prescription 7 and 9-12 groups decreased by 25.8% - 30.9%. Among them, the main peak percentage in the prescription 3 group decreased by only 9.4%, and the stability performance of all the prescriptions was the best. The percentage of the acidic peak and the basic peak of each prescription increased, but in contrast, prescription 3 still performed best, with the increase of 9.1% in the acidic peak (compared with 0h), and the increase of only 0.3% in the basic peak (compared with 0h).

**Table 9. The main peak detection results by WCX for NGF Fc fusion protein formulation**

| Prescription | The percentage of main peak by WCX (%) | | | | |
|---|---|---|---|---|---|
| | 0h | Accelerate for 2 weeks | Changes at acceleration for 2 weeks compared with 0h (Δ) | High-temperature for 2 weeks | Changes at high-temperature for 2 weeks compared with 0h (Δ) |
| 1 | 58.0 | 54.6 | -3.4 | 37.9 | -20.1 |
| 2 | 57.4 | 53.6 | -3.8 | 38.1 | -19.3 |
| 3 | 58.0 | 56.3 | -1.7 | 48.6 | -9.4 |
| 4 | 57.3 | 55.4 | -1.9 | 39.7 | -17.6 |
| 5 | 55.8 | - | - | - | - |
| 6 | 56.2 | - | - | - | - |
| 7 | 52.0 | 40.5 | -11.5 | 23.9 | -28.1 |
| 8 | 56.5 | - | - | - | - |
| 9 | 56.9 | 47.2 | -9.7 | 25.7 | -31.2 |
| 10 | 57.1 | 44.3 | -12.8 | 29.8 | -27.3 |
| 11 | 57.3 | 51.2 | -6.1 | 31.5 | -25.8 |
| 12 | 56.8 | 48.3 | -8.5 | 28.9 | -27.9 |

**Table 10. The acidic peak detection results by WCX for NGF Fc fusion protein formulation**

| Prescription | The percentage of acidic peak by WCX (%) | | | | |
|---|---|---|---|---|---|
| | 0h | Accelerate for 2 weeks | Changes at acceleration for 2 weeks compared with 0h (Δ) | High-temperature for 2 weeks | Changes at high-temperature for 2 weeks compared with 0h (Δ) |
| 1 | 14.8 | 15.5 | 0.7 | 27.1 | 12.3 |
| 2 | 15.5 | 16.9 | 1.4 | 30.1 | 14.6 |
| 3 | 14.6 | 15.3 | 0.7 | 23.7 | 9.1 |
| 4 | 14.1 | 14.5 | 0.4 | 25.4 | 11.3 |
| 5 | 14.5 | - | - | - | - |
| 6 | 14.6 | - | - | - | - |
| 7 | 12.7 | 19.5 | 6.8 | 39.1 | 26.4 |
| 8 | 15.3 | - | - | - | - |
| 9 | 14.5 | 19.2 | 4.7 | 29.3 | 14.8 |
| 10 | 14.6 | 18.9 | 4.3 | 30.1 | 15.5 |
| 11 | 14.2 | 16.8 | 2.6 | 28.5 | 14.3 |
| 12 | 15.1 | 17.6 | 2.5 | 29.3 | 14.2 |

**Table 11. The main peak detection results by WCX for NGF Fc fusion protein formulation**

| Prescription | The percentage of basic peak by WCX (%) | | | | |
|---|---|---|---|---|---|
| | 0h | Accelerate for 2 weeks | Changes at acceleration for 2 weeks compared with 0h (Δ) | High-temperature for 2 weeks | Changes at high-temperature for 2 weeks compared with 0h (Δ) |
| 1 | 27.9 | 20.9 | 3.0 | 36.7 | 8.8 |
| 2 | 27.1 | 19.5 | 2.4 | 31.8 | 4.7 |
| 3 | 27.4 | 18.4 | 1.0 | 27.7 | 0.3 |
| 4 | 28.6 | 20.1 | 1.5 | 24.9 | 6.3 |
| 5 | 29.7 | - | - | - | - |
| 6 | 29.2 | - | - | - | - |
| 7 | 25.2 | 30.0 | 4.8 | 37 | 1.8 |
| 8 | 28.2 | - | - | - | - |
| 9 | 28.6 | 23.6 | 5.0 | 45 | 16.4 |
| 10 | 28.3 | 26.8 | 8.5 | 40.1 | 11.8 |
| 11 | 28.5 | 22.0 | 3.5 | 40 | 11.5 |
| 12 | 28.1 | 24.1 | 6.0 | 41.8 | 13.7 |

According to the above results, compared with other buffers and stabilizers, NGF Fc fusion protein has better stability effect in histidine buffer / trehalose system. Therefore, it can be determined that the most suitable prescription for NGF Fc fusion protein formulation includes the following components: histidine buffer, trehalose, P188 and methionine.

### Example 2. Determination of the prescription of NGF-Fc fusion protein formulation

In order to determine the prescription of NGF-Fc fusion protein formulation, and further verify the stability effect of the above formulation and reflect its advantages, the following experiments were carried out.

### 1. Prescription design and formulation preparation

NGF-Fc fusion protein 2-118-L3Fc10-M3-5 was taken, and samples of each formulation were prepared according to Table 12, and the concentration of fusion protein was 0.1mg/ml.

### 2. Stability test method

The samples just prepared were used as the samples at 0 hous. Subsequently, the samples were taken out under different influencing factors (accelerated 25°C±2°C, high-temperature 40°C±3°C) for two weeks, and the stability of each sample was tested.

The test methods were as follows: the appearance of the solution is observed visually; the osmotic pressure was detected by freezing point osmometer, and the protein concentration was detected by Titer method; the aggregation characteristic of the protein was determined by SEC-HPLC, and the charge heterogeneity was determined by WCX.

**Table 12. The prescription design for NGF-Fc fusion protein**

| Prescription compostions | Prescription | | | | | | | | | |
|---|---|---|---|---|---|---|---|---|---|---|
| | 1 | 2 | 3 | 4 | 5 | 6 | 7 | 8 | 9 | 10 |
| Fusion protein (mg/ml) | 0.1 | 0.1 | 0.5 | 0.1 | 0.1 | 0.5 | 2 | 0.5 | 0.05 | 0.1 |
| L-histidine buffer (mM) | 10 | 20 | 30 | 10 | 20 | 30 | 20 | 20 | 20 | 10 |
| Trehalose (%) | 7 | 9 | 10 | 7 | 9 | 10 | 8 | 10 | 8.5 | 3.8 |
| Methionine (mM) | 1.5 | 1 | 3 | 1.5 | 1 | 3 | 2 | 0.1 | 5 | 5 |
| P188(%) | 0.01 | 0.03 | 0.02 | 0.01 | 0.03 | 0.02 | 0.1 | 0.2 | 0.04 | 0.01 |
| HPBCD (mM) | / | / | / | 5 | 5 | 5 | 6 | 1 | 4 | / |
| Others | / | / | / | / | / | / | / | / | / | NaCl |
| pH value | 5.4 | 5.2 | 5.0 | 5.4 | 5.2 | 5.0 | 5.8 | 6.2 | 5.2 | 5.9 |

### 3. Stability test results

### (1) Osmotic pressure, appearance, protein concentration

At 0 hous, the osmotic pressure of each sample was 285mOsm/kg-310mOsm/kg, and the pH is between 5.15 and 6.28.

After storage for 2 weeks under under accelerated and high-temperature conditions, the sample appearance was observed by naked eyes and it was found that the solutions in prescriptions 1-9 had slight opalescence, but here were no visible insoluble particles. However, a large amount of precipitation appeared in prescription 10, indicating that the formulation could not maintain the stability of NGF Fc fusion protein. Therefore, SEC-HPLC and WCX detection would not be carried out in subsequent experiments.

After storage for 2 weeks under accelerated and high-temperature conditions, the protein concentration of each prescription had no obvious change.

### (2) Aggregate detection

The aggregation of each formulation was detected by SEC-HPLC, the results were shown in Table 13.

According to the results in Table 13, after 2 weeks under accelerated and high-temperature conditions, compared with 0 hous, the main peak percentage by SEC-HPLC of prescriptions 1-9 decreased by relatively smaller amounts, indicating that all prescriptions 1-9 can effectively ensure the stability of the fusion protein. Among them, the main peak percentage of the prescription 4-6 groups decreased by only 0.01% - 0.06% under accelerated conditions after 2 weeks, and decreased by only 0.01% - 0.06% under high-temperature conditions after 2 weeks, demonstrating a relatively better stability effect.

**Table 13. The detection results by SEC-HPLC for NGF-Fc fusion protein formulation**

| Prescription | The percentage of main peak by SEC-HPLC(%) | | | | |
|---|---|---|---|---|---|
| | 0h | Accelerate for 2 weeks | Changes at acceleration for 2 weeks compared with 0h (Δ) | High-temperature for 2 weeks | Changes at high-temperature for 2 weeks compared with 0h (Δ) |
| 1 | 99.15 | 99.01 | -0.14 | 97.85 | -1.30 |
| 2 | 99.42 | 99.32 | -0.10 | 98.41 | -1.01 |
| 3 | 98.98 | 98.59 | -0.39 | 97.60 | -1.38 |
| 4 | 98.92 | 98.93 | 0.01 | 97.99 | -0.93 |
| 5 | 99.66 | 99.64 | -0.02 | 98.89 | -0.77 |
| 6 | 99.35 | 99.29 | -0.06 | 98.51 | -0.84 |
| 7 | 99.25 | 98.89 | -0.36 | 97.78 | -1.47 |
| 8 | 98.97 | 98.54 | -0.43 | 97.59 | -1.38 |
| 9 | 99.03 | 98.89 | -0.14 | 97.67 | -1.36 |
| 10 | 98.05 | - | - | - | - |

### (3) Charge heterogeneity detection

The charge heterogeneity of each prescription formulation was detected by WCX and the results were shown in Tables 14-16.

According to the results in tables 14-16, after 2 weeks under accelerated and high-temperature conditions, compared with 0h, the main peak percentage by WCX in prescriptions 1-9 decreased by less than 13%, and the percentage of acidic peak and basic peak increased by less than 10%, indicating that prescriptions 1-9 can well ensure the stability of the fusion protein. Among them, the main peak percentage of prescriptions 4-6 decreased by only about 8%, showing a relatively better effect.

**Table 14. The main peak detection results by WCX for NGF Fc fusion protein formulation**

| Prescription | The percentage of main peak by WCX (%) | | | | |
|---|---|---|---|---|---|
| | 0h | Accelerate for 2 weeks | Changes at acceleration for 2 weeks compared with 0h (Δ) | High-temperature for 2 weeks | Changes at high-temperature for 2 weeks compared with 0h (Δ) |
| 1 | 57.9 | 55.8 | -2.1 | 46.9 | -11.0 |
| 2 | 58.2 | 56.4 | -1.8 | 48.7 | -9.5 |
| 3 | 56.9 | 55.1 | -1.8 | 45.5 | -11.4 |
| 4 | 58.1 | 57.1 | -1.0 | 49.3 | -8.8 |
| 5 | 57.8 | 57.2 | -0.6 | 49.9 | -7.9 |
| 6 | 57.4 | 56.2 | -1.2 | 49.3 | -8.1 |
| 7 | 57.8 | 55.5 | -2.3 | 45.4 | -12.4 |
| 8 | 52.1 | 49.9 | -2.2 | 41.8 | -10.3 |
| 9 | 54.5 | 53.2 | -1.3 | 41.9 | -12.6 |
| 10 | 50.3 | / | - | - | - |

**Table 15. The acidic peak detection results by WCX for NGF Fc fusion protein formulation**

| Prescription | The percentage of acidic peak by WCX (%) | | | | |
|---|---|---|---|---|---|
| | 0h | Accelerate for 2 weeks | Changes at acceleration for 2 weeks compared with 0h (Δ) | High-temperature for 2 weeks | Changes at high-temperature for 2 weeks compared with 0h (Δ) |
| 1 | 15.4 | 16.2 | 0.8 | 20.5 | 5.1 |
| 2 | 14.9 | 15.8 | 0.9 | 20.9 | 6.0 |
| 3 | 15.1 | 16.3 | 1.2 | 22.9 | 7.8 |
| 4 | 15.2 | 16.1 | 0.9 | 20.5 | 5.3 |
| 5 | 14.8 | 15.6 | 0.8 | 19.8 | 5.0 |
| 6 | 14.9 | 15.8 | 0.9 | 18.9 | 4.0 |
| 7 | 14.7 | 15.8 | 1.1 | 21.5 | 6.8 |
| 8 | 14.6 | 15.8 | 1.2 | 23.6 | 9.0 |
| 9 | 15.1 | 16.3 | 1.2 | 24.1 | 9.0 |
| 10 | 15.7 | - | - | - | - |

**Table 16. The basic peak detection results by WCX for NGF Fc fusion protein formulation**

| Prescription | The percentage of basic peak by WCX (%) | | | | |
|---|---|---|---|---|---|
| | 0h | Accelerate for 2 weeks | Changes at acceleration for 2 weeks compared with 0h (Δ) | High-temperature for 2 weeks | Changes at high-temperature for 2 weeks compared with 0h (Δ) |
| 1 | 26.7 | 28.0 | 1.3 | 32.6 | 5.9 |
| 2 | 26.9 | 27.8 | 0.9 | 30.4 | 3.5 |
| 3 | 28.0 | 28.6 | 0.6 | 31.6 | 3.6 |
| 4 | 26.7 | 26.8 | 0.1 | 30.2 | 3.5 |
| 5 | 27.4 | 27.2 | -0.2 | 30.3 | 2.9 |
| 6 | 27.7 | 28.0 | 0.3 | 31.8 | 4.1 |
| 7 | 27.5 | 28.7 | 1.2 | 33.1 | 5.6 |
| 8 | 33.3 | 34.3 | 1.0 | 34.6 | 1.3 |
| 9 | 30.4 | 30.5 | 0.1 | 34.0 | 3.6 |
| 10 | 34.0 | - | - | - | - |

The above results show that the formulations 1-9 of the present application can well ensure the stability of the fusion protein, especially after adding cyclodextrin, the stability of the fusion protein formulation can be further improved.

### Example 3. Stability validation of the formulation under other conditions

### 1. Preparation of fusion protein formulation

NGF Fc fusion protein was taken, and fusion protein formulation samples were prepared according to the following prescriptions:
Prescription 1:0.1mg/mL 2-118-L3Fc10-M3-5 fusion protein, 20mM L-histidine, 9% trehalose, 1mM methionine, 0.03% poloxamer 188, pH adjusted by acetic acidic is 5.2;
Prescription 2:0.1mg/mL 2-118-L3Fc10-M3-5 fusion protein, 20mM L-histidine, 9% trehalose, 1 mM methionine, 5 mM hydroxypropyl beta cyclodextrin, 0.03% poloxamer 188, pH 5.2 adjusted with acetic acidic;

The samples after preparation were the samples at 0h, after being filtered through a 0.22µm filter membrane, it is divided into 2 ml sterile vials with 1 ml in each vial, plugged and capped.

### 2. Stability detection method

The samples after subpackaging were placed in the stable boxes with different processing conditions of influencing factors, then they were taken out at the time point and tested according to the detection protocol. The conditions were as follows:
Oscillation stability conditions: 2 °C -8 °C, 300 rpm, 2 weeks, 1 month, 2 months;
Freeze thaw stability conditions: -70 °C± 10 °C freeze, 25 °C± 2 °C thaw, repeated freeze-thaw for 5 times;
Illumination stability conditions: 2 °C -8 °C, 4500 ± 500 Lux, 2 weeks, 1 month;
Accelerated stability conditions: 25 °C± 2 °C, 2 weeks, 1 month, 2 months, 3 months, 6 months;
High-temperature stability conditions: 40 °C± 2 °C, 2 weeks, 1 month;
Long-term stability conditions: 2 °C -8 °C, 2 weeks, 1 month, 2 months, 3 months, 6 months.

The test methods were as follows: the appearance of the solution was observed visually; the osmotic pressure was detected by freezing point osmometer, and the protein concentration was detected by Titer method; the aggregation characteristic of the protein was determined by SEC-HPLC, and the charge heterogeneity was determined by WCX.

### 3. Stability test results

### (1) Osmotic pressure, appearance, protein concentration

At 0h, the osmotic pressure of the above samples was 310mosm/kg and 324mosm/kg, respectively, and the pH was 5.35 and 5.36. After placed under different conditions, the osmotic pressure of all samples was maintained in the range of 309mosm/kg and 324mosm/kg, and the pH was 5.29 ± 0.1, which had no obvious change compared with 0 hous.

The above samples were placed in different conditions for a period of time, there was no visible particles produced, and there was no significant change in protein concentration.

### (2) Aggregate detection

The aggregate of each formulation was detected by SEC-HPLC, the results were shown in Tables 17-20.

According to the results in Tables 17-20, it can be seen that at 0 hours, the main peak percentage by SEC-HPLC of the above two samples was 99.45% - 99.69%.

After being placed under oscillation conditions for 2 months, under illumination for 1 month, and after undergoing 5 freeze-thaw cycles, the main peak percentage of both sample groups were approximately 99.41% - 99.71%, which was almost unchanged compared with 0h (Table 17).

After being placed under accelerated conditions for 3 months, the main peak percentage of both sample groups remained around 99%; after accelerated for 6 months, the main peak percentage slightly decreased by 1.70% and 1.20% respectively (Table 18).

After being placed under high-temperature conditions for 2 weeks, the changes of the main peak percentage of both sample groups were only about 1%; as the time continued to extend, the main peak gradually decreased after 1 month of placement, but the decrease was still ≤ 2% (Table 19).

After being placed under the refrigerated conditions for 6 months, the changes of the main peak percentage of both sample groups were all less than 0.5% (Table 20).

The above results show that in terms of aggregate formation, the fusion protein formulation of the present application has excellent stability under various conditions such as oscillation, illumination, freeze-thaw, acceleration for 6 months, high-temperature for 1 month, etc.

**Table 17. The detection results by SEC-HPLC under oscillation, illuminations and freeze-thaw conditions**

| Prescription | The percentage of main peak by SEC-HPLC(%) | | | | | | |
|---|---|---|---|---|---|---|---|
| | 0h | Oscillate for 2 weeks | Oscillate for 1 month | Oscillate for 2 months | Illumination 2 weeks | Illumination for 1 month | Freeze-thaw 5 times |
| 1 | 99.45 | 99.48 | 99.48 | 99.41 | 99.46 | 99.48 | 99.56 |
| 2 | 99.69 | 99.70 | 99.71 | 99.71 | 99.70 | 99.69 | 99.69 |

**Table 18. The detection Results by SEC-HPLC under Accelerated Conditions**

| Prescription | The percentage of main peak by SEC-HPLC(%) | | | | | | |
|---|---|---|---|---|---|---|---|
| | 0h | Accelerate for 2 weeks | Accelerate for 1 month | Accelerate for 2 months | Accelerate for 3 months | Accelerate for 6 months | Changes at 6M compared to 0h (Δ) |
| 1 | 99.45 | 99.48 | 99.41 | 99.26 | 99.11 | 97.75 | -1.70 |
| 2 | 99.69 | 99.5 | 99.51 | 99.18 | 99.00 | 98.49 | -1.20 |

**Table 19. The detection Results by SEC-HPLC under High-temperature Conditions**

| Prescription | The percentage of main peak by SEC-HPLC(%) | | | |
|---|---|---|---|---|
| | 0h | High-temperature for 2 weeks | High-temperature for 1 month | Changes at 1M compared to 0h(Δ) |
| 1 | 99.45 | 98.26 | 97.65 | -1.80 |
| 2 | 99.69 | 98.92 | 98.49 | -1.20 |

**Table 20. The detection Results by SEC-HPLC under long-term refrigeration Conditions**

| Prescription | The percentage of main peak by SEC-HPLC(%) | | | | | | |
|---|---|---|---|---|---|---|---|
| | 0h | Refrigerate for 2 weeks | Refrigerate for 1 month | Refrigerate for 2 months | Refrigerate for 3 months | Refrigerate for 6 months | Changes at 6M compared to 0h 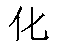 (Δ) |
| 1 | 99.45 | 98.66 | 99.48 | 99.37 | 99.15 | 99.05 | -0.4 |
| 2 | 99.69 | 99.59 | 99.61 | 99.6 | 99.53 | 99.49 | -0.2 |

### (3) Charge heterogeneity detection

The charge heterogeneity of each formulation was detected by WCX and the results were shown in Tables 21-35.

According to the test results in tables 21-35, at 0h, the main peak percentage of both sample groups were 58.1% and 57.7% respectively, the acidic peak percentage was 14.8% and 14.7% respectively, and the basic peak percentage was 27.1% and 27.6% respectively.

After being placed under oscillation conditions for 1 month, the percentages of the main peak, acidicic peak, and basic peak by WCX in both groups showed no significant changes. As the oscillation time increased, after 2 months of oscillation, the main peak percentage by WCX only decreased by 5.3% - 6.2%, the acidic peak percentage only increased by 0.3% - 0.7%, and the basic peak percentage only increased by 5.0% - 5.5% (Tables 21-23).

After the samples were subjected to freeze-thaw treatment 5 times, the percentages of the main peak, acidicic peak and basic peak by WCX in both groups were basically the same as those at 0 hours (Tables 24-26).

After being placed under illumination conditions for 1 month, the main peak percentage by WCX in both groups decreased by only 5.7% - 6.1%, the acidic peak percentage increased by only 1.3% - 1.6%, and the basic peak percentage increased by only 4.5% - 4.6% (Tables 24 - 26).

Under accelerated conditions, with the continuous extension of storage time, the main peak percentage by WCX gradually decreased, and the percentage of the acidic peak and basic peak increased (Table 27-29). Compared with 0h, it decreased by 31.4% after 6 months of acceleration, indicating that the protein charge variable is easy to change when the sample is stored at room temperature for a long time. Therefore, it is necessary to pay close attention to the sample storage temperature in the later stage to avoid sample damage caused by excessive temperature.

Under the high-temperature conditions, as with the continuous extension of storage time, the main peak percentage by WCX gradually decreased, and the percentages of the acidic peak and basic peaks increased (Tables 30-32). The main peak percentage decreased by 22.8% - 23.6%, indicating that the protein samples were damaged at high-temperatures, resulting in a decrease in the main peak of charge variable. Therefore, samples should be stored to avoid damage caused by excessive heat.

After being placed under refrigeration for 6 months, the main peak percentage by WCX in both groups decreased by only 3.6% - 4.0%, and the acidic peak percentage increased by only 0.6% - 0.7%, and the basic peak percentage increased by only 3.0% - 3.3% (Tables 33-35).

**Table 21. The main peak detection results under oscillation conditions**

| Prescription | The main peak percentage by WCX(%) | | | | |
|---|---|---|---|---|---|
| | 0h | Oscillate for 2 weeks | Oscillate for 1 month | Oscillate for 2 months | Changes at 2M compared with 0h (Δ) |
| 1 | 58.1 | 55.7 | 54.5 | 51.9 | -6.2 |
| 2 | 57.7 | 55.3 | 54.5 | 52.4 | -5.3 |

**Table 22. The acidic peak detection results under oscillation conditions**

| Prescription | The acidic peak percentage by WCX (%) | | | | |
|---|---|---|---|---|---|
| | 0h | Oscillate for 2 weeks | Oscillate for 1 month | Oscillate for 2 months | Changes at 2M compared with 0h (Δ) |
| 1 | 14.8 | 15.6 | 15.6 | 15.5 | 0.7 |
| 2 | 14.7 | 15.2 | 15.8 | 15.0 | 0.3 |

**Table 23. The basic peak detection results under oscillation conditions**

| Prescription | The basic peak percentage by WCX (%) | | | | |
|---|---|---|---|---|---|
| | 0h | Oscillate for 2 weeks | Oscillate for 1 month | Oscillate for 2 months | Changes at 2M compared with 0h (Δ) |
| 1 | 27.1 | 28.8 | 29.8 | 32.6 | 5.5 |
| 2 | 27.6 | 29.5 | 29.7 | 32.6 | 5.0 |

**Table 24. The main peak detection results under illumination and Freeze-Thaw conditions**

| Prescription | The main peak percentage by WCX (%) | | | | | |
|---|---|---|---|---|---|---|
| | 0h | Freeze-thaw 5 times | Changes at freeze-thaw 5 times compared with freeze-thaw 0 time | Illumination 2 weeks | Illumination for 1 month | Changes at 1M compared with 0h under illumination conditions (Δ) |
| 1 | 58.1 | 55.7 | -2.4 | 55.5 | 52.0 | -6.1 |
| 2 | 57.7 | 56 | -1.7 | 55.9 | 52.0 | -5.7 |

**Table 25. The acidic peak detection results under illumination and Freeze-Thaw conditions**

| Prescription | The acidic peak percentage by WCX (%) | | | | | |
|---|---|---|---|---|---|---|
| | 0h | Freeze-thaw 5 times | Changes at freeze-thaw 5 times compared with freeze-thaw 0 time | Illumination 2 weeks | Illumination for 1 month | Changes at 1M compared with 0h under illumination conditions (Δ) |
| 1 | 14.8 | 15.5 | 0.7 | 15.7 | 16.4 | 1.6 |
| 2 | 14.7 | 15.3 | 0.6 | 15.5 | 16 | 1.3 |

**Table 26. The basic peak detection results under illumination and Freeze-Thaw conditions**

| Prescription | The basic peak percentage by WCX (%) | | | | | |
|---|---|---|---|---|---|---|
| | 0h | Freeze-thaw 5 times | Changes at freeze-thaw 5 times compared with freeze-thaw 0 time | Illumination 2 weeks | Illumination for 1 month | Changes at 1M compared with 0h under illumination conditions (Δ) |
| 1 | 27.1 | 28.8 | 1.7 | 28.9 | 31.7 | 4.6 |
| 2 | 27.6 | 28.7 | 1.1 | 28.6 | 32.1 | 4.5 |

**Table 27. The main peak detection results under accelerated conditions**

| Prescription | The main peak percentage by WCX (%) | | | | | | |
|---|---|---|---|---|---|---|---|
| | 0h | Accelerate | Accelerate | Accelerate | Accelerate | Accelerate | Changes at |
| | | for 2 weeks | for 1 month | for 2 months | for 3 months | for 6 months | 6M compared with 0h (Δ) |
| 1 | 58.1 | 56.3 | 50.6 | 45.5 | 42.4 | 26.7 | -31.4 |
| 2 | 57.7 | 55.7 | 50.3 | 44.8 | 41.2 | 26.3 | -31.4 |

**Table 28. The acidic peak detection results under accelerated conditions**

| Prescription | The acidic peak percentage by WCX(%) | | | | | | |
|---|---|---|---|---|---|---|---|
| | 0h | Accelerate for 2 weeks | Accelerate for 1 month | Accelerate for 2 months | Accelerate for 3 months | Accelerate for 6 months | Changes at 6M compared with 0h (Δ) |
| 1 | 14.8 | 15.2 | 17.6 | 20.6 | 21 | 40.4 | 25.6 |
| 2 | 14.7 | 15.6 | 17.2 | 20.2 | 21.2 | 39.9 | 25.2 |

**Table 29. The basic peak detection results under accelerated conditions**

| Prescription | The basic peak percentage by WCX(%) | | | | | | |
|---|---|---|---|---|---|---|---|
| | 0h | Accelerate for 2 weeks | Accelerate for 1 month | Accelerate for 2 months | Accelerate for 3 months | Accelerate for 6 months | Changes at 6M compared with 0h (Δ) |
| 1 | 27.1 | 28.5 | 31.9 | 33.9 | 36.5 | 32.9 | 5.8 |
| 2 | 27.6 | 28.7 | 32.5 | 35 | 37.6 | 33.8 | 6.2 |

**Table 30. The main peak detection results under high-temperature conditions**

| Prescription | The main peak percentage by WCX(%) | | | |
|---|---|---|---|---|
| | 0h | High-temperature for 2 weeks | High-temperature for 1 month | Changes at 1M compared with 0h(Δ) |
| 1 | 58.1 | 46.6 | 35.3 | -22.8 |
| 2 | 57.7 | 46.5 | 34.1 | -23.6 |

**Table 31. The acidic peak detection results under high-temperature conditions**

| Prescription | The acidic peak percentage by WCX(%) | | | |
|---|---|---|---|---|
| | 0h | High-temperature for 2 weeks | High-temperature for 1 month | Changes at 1M compared with 0h(Δ) |
| 1 | 14.8 | 24.1 | 31.1 | 16.3 |
| 2 | 14.7 | 24.3 | 31.4 | 16.7 |

**Table 32. The basic peak detection results under high-temperature conditions**

| Prescription | The basic peak percentage by WCX(%) | | | |
|---|---|---|---|---|
| | 0h | High-temperature for 2 weeks | High-temperature for 1 month | Changes at 1M compared with 0h(Δ) |
| 1 | 27.1 | 29.3 | 33.6 | 6.5 |
| 2 | 27.6 | 29.2 | 34.6 | 6.9 |

**Table 33. The main peak detection results under long-term refrigeration conditions**

| Prescription | The main peak percentage by WCX(%) | | | | | | |
|---|---|---|---|---|---|---|---|
| | 0h | Refrigerate for 2 weeks | Refrigerate for 1 month | Refrigerate for 2 months | Refrigerate for 3 months | Refrigerate for 6 months | Changes at 6M compared with 0h (Δ) |
| 1 | 58.1 | 54.6 | 56.1 | 55.4 | 55.7 | 54.5 | -3.6 |
| 2 | 57.7 | 56.5 | 56.3 | 55.1 | 53.9 | 53.7 | -4.0 |

**Table 34. The acidic peak detection results under long-term refrigeration conditions**

| Prescription | The acidic peak percentage by WCX(%) | | | | | | |
|---|---|---|---|---|---|---|---|
| | 0h | Refrigerate for 2 weeks | Refrigerate for 1 month | Refrigerate for 2 months | Refrigerate for 3 months | Refrigerate for 6 months | Changes at 6M compared with 0h (Δ) |
| 1 | 14.8 | 16.3 | 15.3 | 16.3 | 14.9 | 15.4 | 0.6 |
| 2 | 14.7 | 15.1 | 15.4 | 15.8 | 14.4 | 15.4 | 0.7 |

**Table 35. The basic peak detection results under long-term refrigeration conditions**

| Prescription | The basic peak percentage by WCX(%) | | | | | | |
|---|---|---|---|---|---|---|---|
| | 0h | Refrigerate for 2 weeks | Refrigerate for 1 month | Refrigerate for 2 months | Refrigerate for 3 months | Refrigerate for 6 months | Changes at 6M compared with 0h (Δ) |
| 1 | 27.1 | 29.1 | 28.6 | 28.4 | 29.4 | 30.1 | 3.0 |
| 2 | 27.6 | 28.4 | 28.3 | 29.1 | 31.7 | 30.9 | 3.3 |

The above results show that, in terms of charge heterogeneity, the fusion protein formulation of the present application exhibits excellent stability under various conditions such as oscillation, illumination, freeze-thaw, high-temperature, accelerated conditions, and long-term refrigeration. However, it should still be noted that the storage temperature of the formulation should be controlled to avoid high-temperature.

### Example 4. Eye drop prescription screening and validation

In order to ensure that the eye drop is suitable for intraocular administration, a viscosity modifier needs to be added to the formulation to achieve a certain viscosity. Based on the above mentioned determined prescription, a prescription suitable for intraocular administration (such as eye drops) for the NGF-Fc fusion protein was designed and screened.

### I. Screening of viscosity modifiers for eye drop prescription

### 1. Prescription Design and Preparation of Eye Drops

NGF-Fc fusion protein 2-118-L3Fc10-M3-5 was taken, and fusion protein formulations were prepared according to Table 36, wherein the concentration of the fusion protein was 0.1mg/ml. The samples were filtered through a 0.22µm filter membrane and then packaged into disposable blow-fill-seal BFS bottles, with each bottle containing 0.4 mL. Among them, prescription 12 is the NGF eye drop prescription disclosed in the prior art WO2022090339A1, and prescription 13 is obtained by replacing the phosphate buffer in prescription 12 with L-histidine buffer and adjusting the pH to 6.2.

**Table 36. Prescription Design of the NGF-FC Fusion Protein Eye Drop**

| Presc riptio n | Prescription Design | | | | | |
|---|---|---|---|---|---|---|
| | | sodium hyaluron ate (%) | polyviny l alcohol (%) | HPMC (K4M)(%) | polyethylen e glycol 6000(%) | Povidone K30(%) |
| 1 | 20mM Histidine buffer, 8.5% trehalose, 1mM methionine, 5mM HPBCD, 0.03%P188, pH5.2 | 0.01 | | | | |
| 2 | | 0.1 | | | | |
| 3 | | 0.3 | | | | |
| 4 | | | 1 | | | |
| 5 | | | 0.5 | | | |
| 6 | | | | 0.2 | | |
| 7 | | | | 0.2 | 0.5 | |
| 8 | | | 0.5 | 0.2 | | |
| 9 | | | 0.5 | | | 0.5 |
| 10 | | | | | | 2 |
| 11 | | / | / | / | / | / |
| 12 | 0.1g/L fusion protein, 20mM phosphate buffer, 5.4mg/ml trehalose dihydrate, 0.01mg/ml methionine, 10.7mg/ml PEG 6000, 1.07mg/ml HPMC (K4M), 13.1mg/ml mannitol, pH 7.2 | | | | | |
| 13 | 0.1g/L fusion protein, 20mM L-histidine buffer, 5.4mg/ml trehalose dihydrate, 0.01mg/ml methionine, 10.7mg/ml PEG 6000, 1.07mg/ml HPMC (K4M), 13.1mg/ml mannitol, pH 6.2 | | | | | |

### 2. Stability test method

The sample just prepared was used as the sample at 0 hous. Each sample was taken out after 7 days at high-temperature of 40°C±3°C, and the stability index of each sample was tested.

The detection methods were as follows: the appearance of the solution was observed by naked eyes; the aggregation characteristic of the protein was measured by SEC-HPLC; and the charge heterogeneity was measured by WCX.

### 3. Stability test results

At 0 hous, after observing the appearance of the samples, it was found that the samples of prescriptions 1-5 showed obvious white precipitation and the solution became turbid, the samples of prescriptions 8-9 showed a small amount of white flocculent precipitation., and the samples of prescriptions 6-7 and 10-13 showed no visible foreign matters. To improve the detection efficiency and shorten the development time of the prescription development, the samples with precipitation will not be carried out in the later stages of the detection.

The main peak percentages detected by SEC in the other groups were all above 95% at 0 oh. There were no significant differences in the percentages of the main peak, acidicic peak, and basic peak detected by WCX (Table 37).

The viscosity of each sample was measured under the conditions of room temperature of 25°C and shear force of 500 m.s-1. The results were shown in Table 37. Wherein, the prescription 11 was used as the control without adding any viscosity modifier, so the viscosity was not detected. The viscosity of prescriptions 6 and 7was basically the same, indicating that the viscosity of the samples with the simultaneous addition of hydroxypropyl methylcellulose (HPMC K4M) and polyethylene glycol 6000 was not significantly different from that of the samples with only the addition of hydroxypropyl methylcellulose (HPMC K4M). In the prescription 10, polyvinylpyrrolidone (PVP K30) was used as a viscosity modifier with low viscosity, which was not suitable for development as an eye drop.

After being placed under high-temperature conditions for 7 days, the detection results by SEC-HPLC were shown in Table 37: after 7 days of high-temperature conditions, the main peak percentage by SEC in the prescriptions 12 and 13 significantly decreased, falling below 85.0%, while the main peak percentage of the prescriptions 6 and 7 remained above 95.0%, especially prescription 6 exhibited better stability.

Based on the above results, Prescription 6 was selected as the foundation for subsequent formulation development.

**Table 37. Detection Results of Viscosity/Stability for Ophthalmic Solution Formulations**

| Prescr iption | Viscosity (cp) | The main peak percentage by SEC-HPLC(%) | | | WCX | | |
|---|---|---|---|---|---|---|---|
| | | 0h | High-temperatu re for 7 days | Changes at 7D compared with 0h (Δ) | The acidic peak percentage at 0h (%) | The main peak percentage at 0h (%) | The basic peak percentage at 0h(%) |
| 6 | 5.65 | 99.28 | 98.01 | -1.27 | 15.63 | 60.65 | 23.71 |
| 7 | 5.76 | 98.89 | 95.21 | -3.68 | 15.28 | 60.21 | 24.51 |
| 10 | 1.95 | 95.06 | 89.23 | --5.83 | 15.68 | 61.09 | 23.23 |
| 11 | / | 99.32 | 98.25 | -1.07 | 15.51 | 60.29 | 24.20 |
| 12 | 2.58 | 99.19 | 75.23 | -23.96 | 15.20 | 60.30 | 24.50 |
| 13 | 2.34 | 99.03 | 84.25 | -13.78 | 15.12 | 60.75 | 24.13 |

### II. Screening and verification the concentration of the viscosity modifier for the prescription of eye drops

### 1. Prescription design and preparation of eye drops

NGF-Fc fusion protein 2-118-L3Fc10-M3-5 was taken, and f four groups of fusion protein formulation were prepared. Except the viscosity modifier HPMC (K4M) added in different amounts, the other components were the same: 0.1g/mL fusion protein, 20mM histidine buffer, 8.5% trehalose, 1mM methionine, 5mM HPBCD, 0.03%P188, pH adjusted by acetate was 5.2, wherein:
The concentration of HPMC (K4M) added to the prescription 1 was 0.2%; the concentration of HPMC (K4M) added to the prescription 2 was 0.4%; the concentration of HPMC (K4M) added to the prescription 3 was 0.5%; and the concentration of HPMC (K4M) was not added to the prescription 4.

### 2. Stability detection conditions and methods

At 0 hous, the viscosity of each prescription was detected, and prescriptions 1-4 were placed under the following conditions to detect protein content, pH, osmotic pressure and relative biological activity. The biological activity determination method was detected as described in CN103376248A, wherein, the NGF-FC fusion protein without adding excipients was taken as the reference standard, the reference standard was placed under the conditions of -80°C, the biological activity of the samples and the standard were detected each time. The relative biological activity was defined as the ratio of the biological activity of the sample to that of the reference standard. The specification for relative biological activity of the sample is 60%-140% of that of the reference standard.
Oscillation stability conditions: 2°C -8 °C, 300 rpm, 1 week, 1 month;
Freeze thaw stability conditions: -70 °C± 10 °C freeze, 25 °C± 2°C thaw, repeated freeze-thaw for 5 times;
Illumination stability conditions: 2 °C -8 °C, 4500 ± 500 lux, 1 week, 1 month;
Accelerated stability conditions: 25 °C± 2 °C, 1 weeks, 1 month, 2 months, 3 months;
High-temperature stability conditions: 40 °C± 2 °C, 1 week, 1 month;
Long-term stability conditions: 2 °C -8 °C, 1 week, 1 month, 2 months, 3 months.

### 3. Stability detection results

### (1) Appearance, protein concentration, pH

At 0 hous, the viscosity of the samples in prescriptions 1-3 were 5.6cp, 28.7cp and 34.5cp. Prescription 4 was not subjected to viscosity testing since no viscosity modifier was added.

After the samples were placed for a period of time under different influencing factors, there were no visible particles in the solution. The protein content is basically the same as that at 0 hous. In terms of pH, the pH of the samples of each group was between 5.16 and 5.35, all between 5.2±0.2, and the pH of the samples of each group was basically stable.

### (2) stability test results

The stability test results were shown in Tables 38-44.

Osmotic pressure: at 0 hous, the osmotic pressure of the samples was 303mOsm/kg-317mOsm/kg; after being placed under oscillation conditions for 4 weeks (Table 38), under light conditions for 4 weeks (Table 39), and under refrigeration conditions for 12 weeks (Table 43), there was no significant change in the osmotic pressure of each group. After 6 months under acceleration conditions, the osmotic pressure of each group of samples increased 28mOsm/kg-78mOsm/kg (Table 40); after 4 weeks under high-temperature conditions, the osmotic pressure of each group of samples increased 20mOsm/kg-111mOsm/kg (Table 42). The osmotic pressure increases because the coating material of eye drops is semi-permeable, and the water can be lost through the coating material, resulting in the increase of the number of solute particles. The osmotic pressure of solution depends on the number of solute particles in the solution per unit volume, the more solute particles, that is, the higher the osmotic pressure of solution.

Relative biological activity: the relative biological activity of each group of samples was within the standard range after 4 weeks in oscillation conditions (Table 38), 12 weeks in acceleration conditions (Table 41), 4 weeks in high-temperature conditions (Table 42), and 12 weeks in cold storage conditions (Table 44); after 4 weeks in illumination (Table 39), the relative biological activity of each group of samples was reduced to different degrees, indicating that prolonged exposure of eye drops to sunlight can damage the samples and lead to a decrease in activity. Therefor it is advisable to avoid illumination as much as possible in the later stage of sample storage, and the external packaging bag/box can also be used.

**Table 38. Detection results of osmotic pressure and biological activity of eye drop formulations under oscillation Conditions**

| Presc riptio n | Osmotic pressure(mOsm/kg) | | | | Relative biological activity(%) | | | |
|---|---|---|---|---|---|---|---|---|
| | 0h | 1wee k | 1mont h | Changes at 1M compared with 0h (Δ) | 0h | 1week | 1month | Changes at 1M compared with 0h (Δ) |
| 1 | 317 | 313 | 315 | -2 | 101.88 | 88.03 | 95.68 | -6.20 |
| 2 | 303 | 294 | 289 | -14 | 101.82 | 70.41 | 103.04 | 1.22 |
| 3 | 308 | 304 | 313 | 5 | 111.41 | 86.73 | 98.67 | -12.74 |
| 4 | 310 | 305 | 313 | 3 | 100.12 | 93.03 | 96.01 | -4.11 |

**Table 39. Detection results of osmotic pressure and biological activity of eye drop formulations under illumination conditions**

| Presc riptio n | Osmotic pressure(mOsm/kg) | | | | Relative biological activity(%) | | | |
|---|---|---|---|---|---|---|---|---|
| | 0h | 1week | 1mont h | Changes at 1M compared with 0h(Δ) | 0h | 1week | 1month | Changes at 1M compared with 0h(Δ) |
| 1 | 303 | 301 | 305 | 2 | 101.82 | 87.39 | 76.92 | -24.90 |
| 2 | 317 | 302 | 312 | -5 | 101.88 | 94.06 | 59.64 | -42.24 |
| 3 | 308 | 303 | 299 | -9 | 111.41 | 76.56 | 57.56 | -53.85 |
| 4 | 310 | 302 | 315 | 5 | 100.12 | 95.33 | 79.30 | -20.82 |

**Table 40. Detection results of osmotic pressure of eye drop formulations under accelerated conditions**

| Prescri ption | Osmotic pressure (mOsm/kg) | | | | | |
|---|---|---|---|---|---|---|
| | 0h | 1week | 1month | 2 months | 6 months | Changes at 6M compared with 0h (Δ) |
| 1 | 303 | 290 | 314 | 330 | 331 | 28 |
| 2 | 317 | 301 | 329 | 340 | 354 | 37 |
| 3 | 308 | 301 | 315 | 326 | 344 | 36 |
| 4 | 310 | 306 | 313 | 358 | 388 | 78 |

**Table 41. Detection results of biological activity of eye drop formulations under accelerated conditions**

| Prescription | Relative biological activity (%) | | | | | |
|---|---|---|---|---|---|---|
| | 0h | 1week | 1month | 2 months | 6 months | Changes at 6M compared with 0h (Δ) |
| 1 | 101.82 | 70.94 | 88.97 | 105.00 | 83.00 | -18.82 |
| 2 | 101.88 | 94.50 | 90.37 | 83.86 | 78.00 | -23.88 |
| 3 | 111.41 | 86.73 | 92.20 | 74.05 | 74.00 | -37.41 |
| 4 | 100.12 | 94.33 | 104.75 | 94.83 | 72.00 | -28.12 |

**Table 42. Detection results of osmotic pressure and biological activity eye drop formulations under high-temperature conditions**

| Prescription | Osmotic pressure (mOsm/kg) | | | | Relative biological activity (%) | | | |
|---|---|---|---|---|---|---|---|---|
| | 0h | 1week | 1month | Changes at 1M compared with 0h (Δ) | 0h | 1week | 1month | Changes at 1M compared with 0h (Δ) |
| 1 | 303 | 325 | 323 | 20 | 101.82 | 83.56 | 90.30 | -11.52 |
| 2 | 317 | 328 | 336 | 19 | 101.88 | 77.15 | 70.42 | -31.46 |
| 3 | 308 | 309 | 397 | 89 | 111.41 | 73.13 | 67.20 | -44.21 |
| 4 | 310 | 313 | 421 | 111 | 100.12 | 103.09 | 87.98 | -12.14 |

**Table 43. Detection results of ophthalmic of eye drop formulations under long-term refrigeration conditions**

| Prescription | Osmotic pressure (mOsm/kg) | | | | | |
|---|---|---|---|---|---|---|
| | 0h | 1week | 1month | 2 months | 6 months | Changes at 6M compared with 0h (Δ) |
| 1 | 303 | 302 | 308 | 310 | 305 | 2 |
| 2 | 317 | 313 | 313 | 350 | 324 | 7 |
| 3 | 308 | 299 | 310 | 322 | 303 | -5 |
| 4 | 310 | 304 | 305 | 327 | 316 | 6 |

**Table 44. Detection results of biological activity of eye drop formulations under long-term refrigeration conditions**

| Prescription | Relative biological activity(%) | | | | | |
|---|---|---|---|---|---|---|
| | 0h | 1week | 1month | 2 months | 6 months | Changes at 6M compared with 0h(Δ) |
| 1 | 101.82 | 90.42 | 88.97 | 105.60 | 92.00 | -9.82 |
| 2 | 101.88 | 108.99 | 95.75 | 74.62 | 89.00 | -12.88 |
| 3 | 111.41 | 86.34 | 94.12 | 85.09 | 86.00 | -25.41 |
| 4 | 100.12 | 92.71 | 103.41 | 116.11 | 86.00 | -14.12 |

In conclusion, the NGF-Fc fusion protein eye drop formulation of the present application can maintain good stability under conditions such as oscillation, freeze-thawing, acceleration, and long-term refrigeration, maintaining the osmotic pressure of approximately 300 mOsm/kg. The biological activity of the fusion protein was also within the standard range (60%-140%). Especially, the viscosities of prescriptions 2 and 3 were more suitable for eye drops (25cp - 50cp), ensuring that the NGF-Fc fusion protein can remain in the eye for a longer time and exert better effects.

### Example 5. Eye drop prescription stability verification under various conditions

### 1. Stability test method

The samples of prescriptions 2 and 3 in example 4. II were respectively placed in a stabilizer under different influencing factor processing conditions, taken out according to the time point and detected according to the detection scheme. The conditions were as follows:
Oscillation stability conditions: 2 °C -8 °C, 300 rpm, 3 days, 1 week;
Illumination stability conditions (the samples are packed with outer package): 2 °C -8 °C, 4500 ± 500 lux, 1 week, 2 weeks;
Accelerated stability conditions: 25 °C± 2 °C, 1 month;
High-temperature stability conditions: 40 °C± 2 °C, 2 weeks, 1 month;

The detection methods were as follows: the appearance of the solution was observed visually; the protein concentration was detected by Titer method; the aggregation characteristic of the protein was determined by SEC-HPLC, and the charge heterogeneity was determined by iCIEF.

### 3. Stability test results

### (1) Osmotic pressure, appearance, protein concentration

The samples of prescriptions 2 and 3 were placed for a period of time under different factors, there was no visible particles and no significant changes in protein concentration.

### (2) Aggregate detection

The aggregate of each eye drop prescription was detected by SEC-HPLC, the results were shown in Table 45.

According to the results in Table 45, it can be seen that at 0 hours, the main peak percentage of the above two samples by SEC-HPLC was 99.70%-99.80%.

After being placed under oscillation conditions for 7 days, under light conditions for 14 days, and under accelerated conditions (with outer package) for 1 month, the main peaks of the two samples showed no change compared with 0h; after being placed under high-temperature conditions for 1 month, the main peak percentage by SEC-HPLC was 98.80% - 98.90%.

The result indicated that, in terms of the formation of aggregates, the eye drops of the present application exhibited excellent stability under various conditions such as vibration, illumination, acceleration (with outer package), and high-temperature.

**Table 45. Detection results by SEC-HPLC under oscillation, illumination, acceleration and high-temperature conditions**

| Prescription | Main peak percentage by SEC-HPLC(%) | | | | |
|---|---|---|---|---|---|
| | 0h | Oscillate for 1 week | Illumination for 2 weeks | Accelerated for 1 month | High-temperature for 1 month |
| 2 | 99.70 | 99.70 | 99.70 | 99.70 | 98.80 |
| 3 | 99.80 | 99.80 | 99.80 | 99.80 | 98.90 |

### (3) Charge heterogeneity detection

The charge heterogeneity of the eye drop formulation was detect by the iCIEF and the results were shown in Tables 46-51.

According to the results in Tables 46-51, it can be seen that at 0 hours, the main peak percentage of the two groups of samples were 69.15% and 69.67% respectively, the acidic peak percentage were 20.92% and 20.76% respectively, and the basic peak percentage were 9.94% and 9.58% respectively.

After the samples were placed under oscillation conditions for 1 week and under light conditions (with outer package) for 2 weeks, there were no significant changes in the percentages of the main peak, acidicic peak, and basic peak by iCIEF (Tables 46-49).

Under high-temperature conditions, as the storage time increases, the main peak percentage gradually decreased and the acidicic peak percentage increased (Table 50-51). After being placed at high-temperature for 1 month, the main peak percentage decreased by 30.18% - 31.26%. This indicated that the protein samples were damaged under high-temperature, resulting in a decrease in the main peak of charge variables. Therefore, samples should be stored at a safe temperature to avoid sample damage caused by excessive heat.

**Table 46. Detection results of the main peak by iCIEF under oscillation conditions**

| Prescription | Main peak(%) | | | |
|---|---|---|---|---|
| | 0h | Oscillate for 3 days | Oscillate for 1 week | Changes at 1W compared with 0h(Δ) |
| 2 | 69.15 | 69.03 | 68.65 | -0.50 |
| 3 | 69.67 | 70.47 | 68.84 | -0.83 |

**Table 47. Detection results of the acidic peak and basic peak by iCIEF under oscillation conditions**

| Prescription | Acidic peak(%) | | | | Basic peak(%) | | | |
|---|---|---|---|---|---|---|---|---|
| | 0h | Oscillate for 3 days | Oscillate for 1 week | Changes at 1W compared with 0h (Δ) | 0h | Oscillate for 3 days | Oscillate for 1 week | Changes at 1W compared with 0h (Δ) |
| 2 | 20.92 | 21.33 | 21.98 | 1.06 | 9.94 | 9.65 | 9.37 | -0.57 |
| 3 | 20.76 | 20.82 | 21.90 | 1.14 | 9.58 | 8.71 | 9.27 | -0.31 |

**Table 48. Detection results of the main peak by iCIEF under light conditions (with outer package)**

| Prescription | Main peak(%) | | | |
|---|---|---|---|---|
| | 0h | Illumination for 1 week | Illumination for 2 weeks | Changes at 2W compared with 0h(Δ) |
| 2 | 69.15 | 69.06 | 70.06 | 0.91 |
| 3 | 69.67 | 69.98 | 70.16 | 0.49 |

**Table 49. Detection results of the acidic peak and the basic peak by iCIEF under light conditions (with outer package)**

| Prescription | Acidic peak(%) | | | | Basic peak(%) | | | |
|---|---|---|---|---|---|---|---|---|
| | 0h | Illumination for 1 week | Illumination for 2 weeks | Changes at 2W compared with 0h (Δ) | 0h | Illumination for 1 week | Illumination for 2 weeks | Changes at 2W compared with 0h (Δ) |
| 2 | 20.92 | 21.44 | 20.36 | -0.56 | 9.94 | 9.51 | 9.59 | -0.35 |
| 3 | 20.76 | 20.68 | 20.24 | -0.52 | 9.58 | 9.34 | 9.61 | 0.03 |

**Table 50. Detection results of the main peak by iCIEF under high-temperature conditions**

| Prescription | Main peak(%) | | | |
|---|---|---|---|---|
| | 0h | High-temperature for 2 weeks | High-temperature for 1 month | Changes at 1W compared with 0h (Δ) |
| 2 | 69.15 | 50.99 | 37.89 | -31.26 |
| 3 | 69.67 | 52.16 | 39.49 | -30.18 |

**Table 51. Detection results of the acidic peak and the basic peak by iCIEF under high-temperature conditions**

| Prescription | Acidic peak(%) | | | | Basic peak(%) | | | |
|---|---|---|---|---|---|---|---|---|
| | 0h | High-temperature for 2 weeks | High-temperature for 1 month | Changes at 1W compared with 0h (Δ) | 0h | High-temperature for 2 weeks | High-temperature for 1 month | Changes at 1W compared with 0h (Δ) |
| 2 | 20.92 | 38.44 | 52.91 | 31.99 | 9.94 | 10.57 | 9.22 | -0.72 |
| 3 | 20.76 | 37.47 | 51.53 | 30.77 | 9.58 | 10.37 | 9.98 | 0.40 |

The above results indicated that, in terms of protein aggregation and charge heterogeneity, the eye drops of the present application exhibited excellent stability under various conditions such as oscillation, illumination, acceleration, and high-temperature. However, it should still be noted that the temperature of the stored formulation should be controlled to avoid high-temperatures.

### Sequence list

SEQ ID NO: 1 (Amino acidic sequence of 2-118-L3Fc10-M3-5) 
SSSHPIFHRGEESVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVRGGGGSGGGGSGGGGSDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQDWLN
GKEYKCKVSNKALPASIEKTISKAKGQPREPQVYTLPPSREEMTKNQVSLTCLVKGFY
PSDIAVEWESNGQPENNYKTTPPVLDSDGSFFLYSKLTVDKSRWQQGNVFSCSVMHE
ALHNHYTQKSLSLSPGK
SEQ ID NO: 2 (mature β-NGF) 
SSSHPIFHRGEESVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVR
SEQ ID NO: 3 (linker)
   GGGGSGGGGSGGGGS
SEQ ID NO: 4 (Fc moiety)

DKTHTCPPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPASIEK
TISKAKGQPREPQVYTLPPSREEMTKNQVSLTCLVKGFYPSDIAVEWESNGQPENNYK
TTPPVLDSDGSFFLYSKLTVDKSRWQQGNVFSCSVMHEALHNHYTQKSLSLSPGK

## Claims

1. An NGF-Fc fusion protein formulation, wherein the formulation comprises an NGF-Fc fusion protein, a stabilizer, a surfactant, an antioxidant and a buffer, wherein, the stabilizer is trehalose or sucrose, the antioxidant is methionine, the surfactant is poloxamer, and the stabilizer is histidine buffer.

2. The NGF-Fc fusion protein formulation of claim 1, wherein the concentration of the fusion protein is from 0.05mg/ml to 5.0mg/ml, preferably from 0.05mg/ml to 2.0mg/ml, and more preferably from 0.1mg/ml to 0.5mg/ml.

3. The NGF-Fc fusion protein formulation of claim 1 or 2, wherein the concentration of the histidine buffer is from 8mM to 50mM, preferably from 10mM to 30mM.

4. The NGF-Fc fusion protein formulation of any one of claims 1-3, wherein the concentration of the sucrose or the trehalose is from 6% to 12%, preferably from 7% to 10%.

5. The NGF-Fc fusion protein formulation of any one of claims 1-4, wherein the concentration of the methionine is from 0.05mM to 7mM, preferably from 0.1mM to 5mM, and more preferably from 1mM to 3mM.

6. The NGF-Fc fusion protein formulation of any one of claims 1-5, wherein the concentration of the poloxamer is from 0.005% to 0.3%, preferably 0.01% to 0.2%, and more preferably 0.01% to 0.03%.

7. The NGF-Fc fusion protein formulation of any one of claims 1-6, wherein the pH of the formulation is from 5.0 to 6.5, preferably from 5.0 to 6.2, and more preferably from 5.0 to 5.4.

8. The NGF-Fc fusion protein formulation of any one of claims 1-7, wherein the formulation further comprises a cyclodextrin, preferably the cyclodextrin is hydroxypropyl beta cyclodextrin.

9. The NGF-Fc fusion protein formulation of claim 8, wherein the concentration of the cyclodextrin is from 0.05mM to 8mM, preferably from 1mM to 6mM, and more preferably 5 mM.

10. The NGF-Fc fusion protein formulation of claim 1, wherein the formulation is any one of the following formulations:
(1) the concentration of the fusion protein is 0.05mg/ml, 0.06mg/ml, 0.08mg/ml, 0.09mg/ml, 0.10mg/ml, 0.11mg/ml, 0.12mg/ml, 0.14mg/ml, 0.16mg/ml, 0.18mg/ml or 2.0mg/ml, the concentration of the histidine buffer is from 10mM to 30mM, the concentration of the sucrose or the trehalose is from 7% to 10%, the concentration of the methionine is from 0.1mM to 5Mm, the concentration of the poloxamer is from 0.01% to 0.2%, and the pH of the formulation is from 5.0 to 6.2;
(2) the concentration of the fusion protein is from 0.05mg/ml to 2.0mg/ml, the concentration of the histidine buffer is 10mM, 15mM, 20mM, 25mM or 30mM, the concentration of the sucrose or the trehalose is from 7% to 10%, the concentration of the methionine is from 0.1mM to 5Mm, the concentration of the poloxamer is from 0.01% to 0.2%, and the pH of the formulation is from 5.0 to 6.2;
(3) the concentration of the fusion protein is from 0.05mg/ml to 2.0mg/ml, the concentration of the histidine buffer is from 10mM to 30mM, the concentration of the sucrose or the trehalose is 7%, 7.5%, 8%, 8.5%, 9%, 9.5% or 10%, the concentration of the methionine is from 0.1mM to 5Mm, the concentration of the poloxamer is from 0.01% to 0.2%, and the pH of the formulation is from 5.0 to 6.2;
(4) the concentration of the fusion protein is from 0.05mg/ml to 2.0mg/ml, the concentration of the histidine buffer is from 10mM to 30mM, the concentration of the sucrose or the trehalose is from 7% to 10%, the concentration of the methionine is 0.1mM, 0.5mM, 1mM, 1.5mM, 2mM, 2.5mM, 3mM, 3.5mM, 4mM, 4.5mM or 5mM, the concentration of the poloxamer is from 0.01% to 0.2%, and the pH of the formulation is from 5.0 to 6.2;
(5) the concentration of the fusion protein is from 0.05mg/ml to 2.0mg/ml, the concentration of the histidine buffer is from 10mM to 30mM, the concentration of the sucrose or the trehalose is from 7% to 10%, the concentration of the methionine is from 0.1mM to 5mM, the concentration of the poloxamer is 0.01%, 0.02%, 0.03%, 0.04%, 0.06%, 0.08%, 0.1%, 0.12%, 0.14%, 0.16%, 0.18% or 0.2%, and the pH of the formulation is from 5.0 to 6.2;
(6) the concentration of the fusion protein is from 0.05mg/ml to 2.0mg/ml, the concentration of the histidine buffer is from 10mM to 30mM, the concentration of the sucrose or the trehalose is from 7% to 10%, the concentration of the methionine is from 0.1mM to 5mM, the concentration of the poloxamer is from 0.01% to 0.2%, and the pH of the formulation is 5.0, 5.1, 5.2, 5.3, 5.4, 5.6, 5.8, 6.0 or 6.2.

11. The NGF-Fc fusion protein formulation of claim 1, wherein the formulation is any one of the following formulations:
(1) the concentration of the fusion protein is 0.1mg/ml, the concentration of the histidine buffer is 10mM, the concentration of the sucrose or the trehalose is 7%, the concentration of the methionine is 1.5mM, the concentration of the poloxamer is 0.01%, and the pH of the formulation is 5.4;
(2) the concentration of the fusion protein is 0.1mg/ml, the concentration of the histidine buffer is 20mM, the concentration of the sucrose or the trehalose is 9%, the concentration of the methionine is 1.0mM, the concentration of the poloxamer is 0.03%, and the pH of the formulation is 5.2;
(3) the concentration of the fusion protein is 0.5mg/ml, the concentration of the histidine buffer is 30mM, the concentration of the sucrose or the trehalose is 10%, the concentration of the methionine is 3mM, the concentration of the poloxamer is 0.02%, and the pH of the formulation is 5.0;
(4) the concentration of the fusion protein is 2.0mg/ml, the concentration of the histidine buffer is 20mM, the concentration of the sucrose or the trehalose is 8%, the concentration of the methionine is 2.0mM, the concentration of the poloxamer is 0.1%, and the pH of the formulation is 5.8;
(5) the concentration of the fusion protein is 0.5mg/ml, the concentration of the histidine buffer is 20mM, the concentration of the sucrose or the trehalose is 10%, the concentration of the methionine is 0.1mM, the concentration of the poloxamer is 0.2%, and the pH of the formulation is 6.2;
(6) the concentration of the fusion protein is 0.05mg/ml, the concentration of the histidine buffer is 20mM, the concentration of the sucrose or the trehalose is 8.5%, the concentration of the methionine is 5.0mM, the concentration of the poloxamer is 0.04%, and the pH of the formulation is 5.2.

12. The NGF-Fc fusion protein formulation of any one of claims 1-11, wherein the concentration of the cyclodextrin is 1 mM, 2 mM, 3 mM, 4 mM, 5 mM or 6 mM.

13. The NGF-Fc fusion protein formulation of any one of claims 1-12, wherein the formulation is a liquid formulation, and preferably the formulation is an injection formulation or an eye drop formulation.

14. The NGF-Fc fusion protein formulation of claim 13, wherein the formulation is an eye drop formulation, and further comprises a viscosity modifier.

15. The NGF-Fc fusion protein formulation of claim 14, wherein the viscosity modifier is hydroxypropyl methylcellulose; more preferably, the viscosity modifier is hydroxypropyl methylcellulose K4M.

16. The NGF-Fc fusion protein formulation of claim15, wherein the concentration of the viscosity modifier is from 0.4% to 0.5%.

17. The NGF-Fc fusion protein formulation of claim15, wherein the eye drop formulation comprises the following components: the concentration of the fusion protein is 0.1mg/ml, the concentration of the histidine buffer is 20mM, the concentration of the trehalose is 8.5%, the concentration of the methionine is 1.0mM, the concentration of the poloxamer is 0.03%, the concentration of the cyclodextrin is 5mM, the concentration of the hydroxypropyl methylcellulose is 0.4%, 0.45% or 0.5%, and the pH of the formulation is 5.2.

18. The NGF-Fc fusion protein formulation of any one of claims 1-17, wherein the NGF-Fc fusion protein comprises an NGF moiety and an Fc moiety from N-terminus to C-terminus, the Fc moiety is derived from an Fc moiety of IgG or a mutant of an Fc moiety of IgG, and the mutant of the Fc moiety of IgG comprises a site mutation associated with ADCC/CDC activity.

19. The NGF-Fc fusion protein formulation of claim18, wherein the NGF moiety is a human nerve growth factor, preferably a human nerve growth factor mutant.

20. The NGF-Fc fusion protein formulation of claim19, wherein the human nerve growth factor mutant comprises the mutation site of Phe12Glu with reference to the amino acidic position set forth in wild-type human nerve growth factor; preferably, the human nerve growth factor comprises the amino acidic sequence of SEQ ID NO:2.

21. The NGF-Fc fusion protein formulation of any one of claims 18-20, wherein the NGF moiety is fused to the Fc moiety via a polypeptide linker, the polypeptide linker comprises the amino acidic sequence of SEQ ID NO:3.

22. The NGF-Fc fusion protein formulation of any one of claims 18-21, wherein the Fc moiety is derived from an Fc of IgG1 comprising the amino acidic sequence of SEQ ID NO:4.

23. The NGF-Fc fusion protein formulation of any one of claims 18-22, wherein the NGF-Fc fusion protein comprises the amino acidic sequence of SEQ ID NO:1.

24. The NGF-Fc fusion protein formulation of any one of claims 1-23, wherein the NGF-Fc fusion protein formulation is used for the preparation of a medicament for the treatment of NGF-related diseases.

25. The use of claim 24, wherein the NGF-related diseases is a neurological disease; preferably, the neurological disease is selected from the group consisting of: neonatal hypoxic-ischemic encephalopathy, cerebral palsy, critical illness myopathy, nerve deafness, recurrent laryngeal nerve injury, traumatic brain injury, tooth nerve injury, cerebral stroke, Down syndrome, amyotrophic lateral sclerosis, multiple sclerosis, spinal muscular atrophy, diffuse brain injury, thymus dysplasia, optic contusion, follicular dysplasia, spinal cord injury, glaucoma, neurotrophic keratitis, optic injury, neuromyelitis optica, retinal associated diseases, urinary incontinence, Alzheimer's disease, Parkinson's disease, Huntington's disease, dementia, Hypertensive Intracerebral Hemorrhage Neurological Dysfunction, Cerebral Small Vessel Disease, Acute Ischemic Stroke, corneal endothelial dystrophy, diabetic neuropathy, diabetic foot ulcer, neurogenic skin ulcer, pressure sore, neurotrophic corneal ulcer, diabetic corneal ulcer, and macular hole.

26. The method of claim 24, wherein the NGF-related disease is a non-neurological disease; preferably, the non-neurological disease is selected from the group consisting of: atrophy of spleen, contusion of spleen, diminished ovarian reserve, premature ovarian failure, Ovarian Hyperstimulation Syndrome, ovarian remnant syndrome, ovarian follicular dysplasia, spermatogenesis disorder such as oligozoospermia, asthenospermia, and oligoasthenospermia, ischemic ulcer, stress ulcer, rheumatoid ulcer, liver fibrosis, corneal ulcer, burns, oral ulcer, and venous leg ulcers.
